# EUROPEAN PATENT APPLICATION

(11) **EP 4 834 214 A1**
(43) Date of publication of application: **04.11.2026**
(21) Application number: 23963011.4
(22) Date of filing: 25.12.2023
(51) Int. Cl.: A61B 5/0538

(54) **MEDICAL DEVICE WITH SENSOR**

(71) Applicant: ASAHI INTECC CO., LTD., Seto-shi, Aichi, 489-0071 (JP)
(72) Inventor: NAMIMA, Satoshi, Seto-shi, Aichi 489-0071 (JP)
(74) Representative: Prinz & Partner mbB
(86) International application number: PCT/JP2023/046450
(87) International publication number: WO 2025/141650

(57) **Abstract**

A medical device with a sensor comprises a sensor, wiring electrically connected to the sensor, a conductive first tube located on the more proximal end side than the sensor and electrically connected to the wiring, a conductive second tube located on the more proximal end side than the first tube and electrically connected to the wiring, and an insulator which is sandwiched between the first tube and the second tube, a part of which is externally exposed, and the shape of the exposed portion of which when viewed from a first radial direction is different from the shape of the exposed portion of which when viewed from a second radial direction which is different from the first radial direction.

## Description

### TECHNICAL FIELD

The present disclosure relates to a medical device with a sensor.

### BACKGROUND ART

Medical devices with sensors being attached to their distal end portions are known. For example, in Patent Literatures 1 and 2, a device having a sensor, and a connector in which conductive and insulating portions are arranged alternately is disclosed. In Patent Literature 3, a multi-lumen catheter in which a thermocouple serving as a temperature sensor is inserted into a fourth lumen is disclosed. Hereinafter, the insulating portion will also be referred to as an "insulator."

### Citation List

### Patent Literature

[Patent Literature 1] U.S. Pat. No. 9878142
[Patent Literature 2] JP 2016-527963 W
[Patent Literature 3] JP 2009-254423 A

### SUMMARY OF THE INVENTION

### TECHNICAL PROBLEM

In the device described in Patent Literatures 1 and 2, due to the significant difference in hardness between the conductive portion and the insulator, stress can be concentrated at the joint part between the conductive portion and the insulator when the connector is connected to an external device, resulting in the breakage of the less rigid insulator than the conductive portion. In Patent Literature 3, the structure of the connector is not considered at all.

The present disclosure was made to solve at least a part of the problems mentioned above and aims to inhibit the breakage of an insulator in a medical device with a sensor.

### SOLUTION TO PROBLEM

The present disclosure was made to solve at least a part of the problems mentioned above, and can be implemented as the following aspects.
(1) According to one aspect of the present disclosure, a medical device with a sensor is provided. The medical device with a sensor comprises a sensor, wiring electrically connected to the sensor, a conductive first tube located on the more proximal end side than the sensor and electrically connected to the wiring, a conductive second tube located on the more proximal end side than the first tube and electrically connected to the wiring, and an insulator which is sandwiched between the first tube and the second tube, a part of which is externally exposed, and the shape of the exposed portion of which when viewed from a first radial direction is different from the shape of the exposed portion of which when viewed from a second radial direction different from the first radial direction.
   According to this configuration, as for the insulator, the shape of the exposed portion when viewed from the first radial direction is different from the shape of the exposed portion when viewed from the second radial direction that is different from the first radial direction. In other words, the insulator does not have a right cylindrical shape. Therefore, even if bending stress acts in the vicinity of the insulator when the connector is connected to an external device, stress is less likely to concentrate on the insulator. As a result, the breakage of the insulator can be prevented.
(2) In the medical device with a sensor of the above aspect, the insulator may be an adhesive. According to this configuration, the connector including the first and second tubes and the insulator can be easily manufactured.
(3) In the medical device with a sensor of the above aspect, the proximal end of the first tube may be located on the more proximal end side than the distal end of the second tube. According to this configuration, as stress on the insulator is distributed more evenly, damage to the insulator is prevented more effectively.
(4) In the medical device with a sensor of the above aspect, a virtual cylindrical surface formed by extending the outer peripheral surface of the first tube to the proximal end side substantially may coincide with the outer peripheral surface of the second tube. According to this configuration, the first and second tubes can be configured to be like a single tube.
(5) In the medical device with a sensor of the above aspect, it may further comprise a core shaft that passes through the insulator. According to this configuration, the strength of a portion where the insulator is positioned can be improved in the connector. As a result, the breakage of the insulator can be inhibited still further to improve safety.
(6) In the medical device with a sensor of the above aspect, at least any one of a proximal end side end face of the first tube and a distal end side end face of the second tube may include a portion inclined with respect to the long axis direction of the medical device with a sensor. According to this configuration, even if bending stress acts in the vicinity of the insulator when the connector is connected to an external device, stress on the insulator is distributed more evenly, thereby preventing damage to the insulator more effectively.
(7) In the medical device with a sensor of the above aspect, at least a part of the proximal end side end face of the first tube may be substantially parallel to at least a part of the distal end side end face of the second tube. According to this configuration, a portion consisting solely of the insulator disappears in the longitudinal direction. For this reason, stress on the insulator is distributed more evenly, thereby preventing damage to the insulator more effectively.
(8) In the medical device with a sensor of the above aspect, at least any one of the first tube and the second tube may be joined to the insulator at the inner peripheral surface. According to this configuration, because the joint strength between the first and second tubes and the insulator can be improved, the connector that is even less prone to break can be provided.
(9) In the medical device with a sensor of the above aspect, the insulator sandwiched between the first tube and the second tube is a first insulator, and it may further comprise a conductive third tube located on the more proximal end side than the second tube and electrically connected to the wiring, and a second insulator which is sandwiched between the second tube and the third tube, a part of which is externally exposed, and the shape of the exposed portion of which when viewed from the first radial direction is different from the shape of the exposed portion of which when viewed from the second radial direction. According to this configuration, since the first and second insulators do not have right cylindrical shapes, the breakage of the first and second insulators can be inhibited.
(10) In the medical device with a sensor of the above aspect, the shape of the exposed portion of the first insulator may be substantially identical with the shape of the exposed portion of the second insulator when viewed from the first radial direction. According to this configuration, it is possible to easily manufacture the medical device with a sensor that can inhibit the breakage of the insulators.

The present disclosure may be realized in various aspects; it can be realized with aspects such as a medical device with a sensor, a connector part for the medical device with a sensor, and a catheter including the medical device with a sensor, as well as a method for manufacturing the same, for example.

### BRIEF DESCRIPTION OF THE DRAWINGS

Fig. 1 is an explanatory view illustrating the configuration of a medical device with a sensor.
Fig. 2 is an enlarged sectional view of the distal end side of the medical device with a sensor.
Fig. 3 is an explanatory view illustrating the configuration of a tubular member.
Fig. 4 is an explanatory view illustrating the configuration of a sensor sheet.
Fig. 5 is an explanatory view explaining a method for making a sensor.
Fig. 6 is an explanatory view illustrating the configuration of wiring.
Fig. 7 is an enlarged view of the proximal end side in the medical device with a sensor.
Fig. 8 is an explanatory view illustrating the configuration of a connector.
Fig. 9 is an explanatory view illustrating the configuration of the proximal end portion in the wiring.
Fig. 10 is a transverse sectional view of the connector along the line A1-A1 of Fig. 8.
Fig. 11 is a transverse sectional view of the connector along the line A2-A2 of Fig. 8.
Fig. 12 is a longitudinal sectional view of the connector along line B-B of Fig. 10.
Fig. 13 is a view explaining the relationship between a first tube and a second tube.
Fig. 14 is an external view of the connector when viewed from a first radial direction D1.
Fig. 15 is an external view of the connector when viewed from a second radial direction D2.
Fig. 16 is an external view of a connector of a second embodiment when viewed from the second radial direction D2.
Fig. 17 is an external view of a connector of a third embodiment when viewed from the second radial direction D2.
Fig. 18 is an enlarged view of the proximal end side of a medical device with a sensor in a fourth embodiment.
Fig. 19 is a longitudinal sectional view of a connector in a fifth embodiment.
Fig. 20 is an enlarged view of the proximal end side of a medical device with a sensor in a sixth embodiment.

### DESCRIPTION OF EMBODIMENTS

### <First Embodiment>

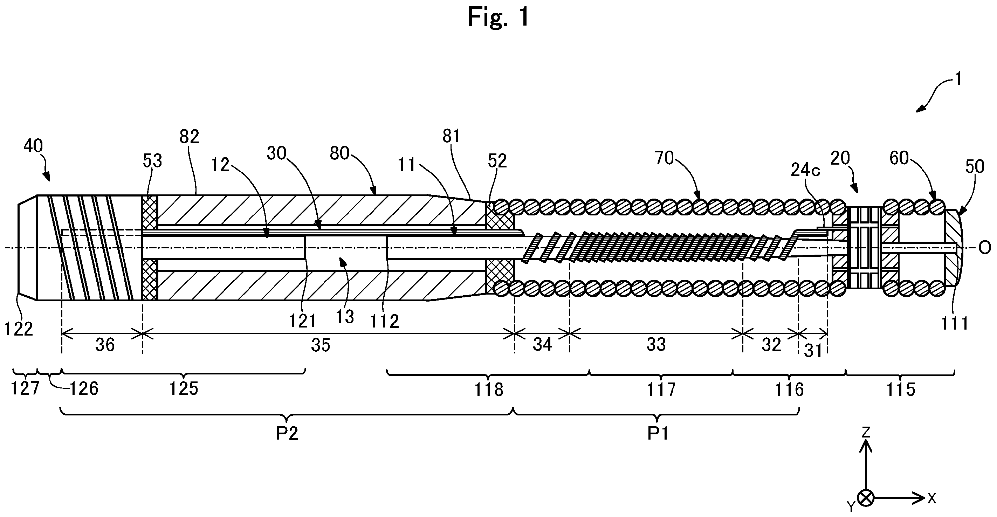

Fig. 1 is an explanatory view illustrating the configuration of a medical device with a sensor 1. Fig. 1 illustrates a sensor 20 and a connector 40 in the external configurations, and other portions in the longitudinal sectional configurations. The medical device with a sensor 1 is a device which is inserted into and used in a cerebral blood vessel to measure the electrical resistance of bodily fluids such as blood flowing through the vessel, for example. The medical device with a sensor 1 is a guide wire with a sensor. The electrical resistance detected by the medical device with a sensor 1 is used, for example, to determine the type of a blood clot generated in the cerebral blood vessel.

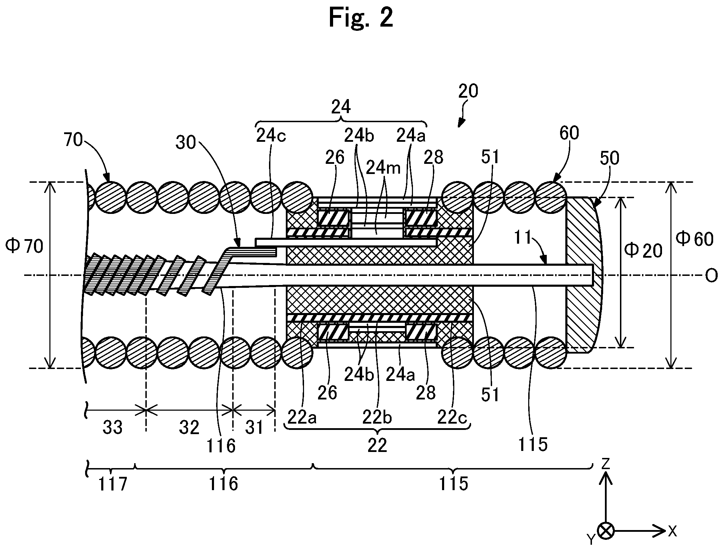

In Fig. 1, the axis passing through the center of the medical device with a sensor 1 is represented by the axis line O. Hereinafter, for each member in the medical device with a sensor 1, the center axis at least passing through the centers of a first core shaft 11, a second core shaft 12, a first coil 60, a second coil 70, a tube 80, and first to sixth tubes 41a-41f will be described as coinciding with the axis line O. The center axis passing through the center of each member in the medical device with a sensor 1 may differ from the axis line O. Fig. 1 illustrates XYZ-axes that are orthogonal to each other. The X-axis corresponds to the longitudinal direction of the medical device with a sensor 1; in other words, it corresponds to the long axis direction of the medical device with a sensor 1. The Y-axis corresponds to the width direction of the medical device with a sensor 1. The Z-axis corresponds to the height direction of the medical device with a sensor 1. The +X-axis direction in Fig. 1 is referred to as the "distal end side" of the medical device with a sensor 1 and each component member. The -X-axis direction in Fig. 1 is referred to as the "proximal end side" of the medical device with a sensor 1 and each component member. With regard to the medical device with a sensor 1 and each component member, one end located on the distal end side is referred to as the "distal end," and the other end located on the proximal end side is referred to as the "proximal end." For the medical device with a sensor 1 and each component member, the distal end and its vicinity are referred to as the "distal end portion," and the proximal end and its vicinity are referred to as the "proximal end portion." The distal end side is inserted into a living body, and the proximal end side is operated by an operator such as a doctor. These points are also common to Fig. 2 and subsequent figures. In the present embodiment, expressions "the same" and "equal" indicate much the same, and allow variances resulting from manufacturing tolerances or the like. In the present embodiment, the expression "approximately constant" indicates "nearly constant," and allows variances resulting from manufacturing tolerances or the like.

The medical device with a sensor 1 comprises the first core shaft 11, the second core shaft 12, a sensor 20, wiring 30, a connector 40, a distal tip 50, the first coil 60, the second coil 70, and the tube 80.

The first core shaft 11 is a solid member which has an elongated shape extending along the X-axis direction. The first core shaft 11 has, in order from a distal end 111 toward a proximal end 112, a first portion 115, a second portion 116, a third portion 117, and a fourth portion 118. The outer peripheral surface of the first core shaft 11 is coated by a first insulating tube 15 which has insulation properties. In other words, the first core shaft 11 includes the first insulating tube 15.

The first portion 115 is a portion provided on the most distal end side of the first core shaft 11. The first portion 115 is a portion where press processes have been applied to the smallest outer diameter portion in the first core shaft 11. The number of press processes and the extent of presses with respect to the first portion 115 may be determined optionally. The second portion 116 is a portion provided between the first portion 115 and the third portion 117. The distal end of the second portion 116 has the same outer diameter as that of the proximal end of the first portion 115. The proximal end of the second portion 116 has the same outer diameter as that of the distal end of the third portion 117. The second portion 116 has a gradually tapered shape which gradually decreases in outer diameter from the proximal end toward the distal end. The third portion 117 is a portion provided between the second portion 116 and the fourth portion 118. The distal end of the third portion 117 has the same outer diameter as that of the proximal end of the second portion 116. The proximal end of the third portion 117 has the same outer diameter as that of the distal end of the fourth portion 118. The third portion 117 has a gradually tapered shape which gradually decreases in outer diameter from the proximal end toward the distal end. The inclination of the taper in the third portion 117 is greater than that of the taper in the second portion 116. The second portion 116 is a tapered portion. The third portion 117 is a tapered portion. The fourth portion 118 is a portion provided on the most proximal end side of the first core shaft 11. The fourth portion 118 is a portion having the largest outer diameter in the first core shaft 11, and has a substantially cylindrical shape with a substantially constant outer diameter. The outer diameters, the lengths in the axis line O direction, and the transverse sectional shapes of the first, second, third, and fourth portions 115, 116, 117, 118 may be determined optionally.

The second core shaft 12 is a solid member which has an elongated shape extending along the X-axis direction. The second core shaft 12 is positioned on the more proximal end side than and coaxially with the first core shaft 11. A gap 13 is formed between the first core shaft 11 and the second core shaft 12. The gap 13 is formed with the proximal end face of the first core shaft 11 and the distal end face of the second core shaft 12 being positioned apart from one another. The second core shaft 12 has, in order from a distal end 121 toward a proximal end 122, a fifth portion 125, a sixth portion 126, and a seventh portion 127. The outer peripheral surface of the second core shaft 12 is coated by a second insulating tube 16 which has insulation properties. In other words, the second core shaft 12 includes the second insulating tube 16.

The fifth portion 125 is a portion provided on the most distal end side of the second core shaft 12. The fifth portion 125 is a portion having the least outer diameter in the second core shaft 12, and has a roughly cylindrical shape with a substantially constant outer diameter. The sixth portion 126 is a portion provided between the fifth portion 125 and the sixth portion 126. The distal end of the sixth portion 126 has the same outer diameter as that of the proximal end of the fifth portion 125. The proximal end of the sixth portion 126 has the same outer diameter as that of the distal end of the seventh portion 127. The sixth portion 126 has a gradually tapered shape which gradually decreases in outer diameter from the proximal end toward the distal end. The seventh portion 127 is a portion provided on the most proximal end side of the second core shaft 12. The seventh portion 127 has a gradually tapered shape which gradually expands in outer diameter from the proximal end toward the distal end. The seventh portion 127 is externally exposed from the proximal end of the connector 40. The outer diameters, the lengths in the axis line O direction, and the transverse sectional shapes of the fifth, sixth and seventh portions 125, 126 and 127 may be determined optionally.

The first coil 60 and the second coil 70 are cylindrical coils which have flexibility and have substantially constant outer diameters from the proximal ends toward the distal ends. The first coil 60 and the second coil 70 are multi-thread coils formed by a plurality of wires wound in a multi-thread manner. The first coil 60 is positioned on the more distal end side than the sensor 20. The first coil 60 surrounds the outer periphery of the first portion 115 of the first core shaft 11 which protrudes from the sensor 20 toward the distal end side. The second coil 70 is positioned on the more proximal end side than the sensor 20. The second coil 70 surrounds the outer peripheries of the second portion 116, third portion 117 and a portion of the distal end side of the fourth portion 118 in the first core shaft 11. The first coil 60 and the second coil 70 can be formed from any material. The first coil 60 and the second coil 70 can be formed by using at least any one of stainless steel alloys, superelastic alloys, radiolucent alloys, and radiopaque alloys. As stainless steel alloys, SUS 304 and SUS 316 can be exemplified. As superelastic alloys, nickel-titanium and nickel-titanium alloys can be exemplified. As radiolucent alloys, piano wire, nickel-chromium based alloys, and cobalt alloys can be exemplified. As radiopaque alloys, gold, platinum, tungsten, and alloys containing these elements can be exemplified.

The first coil 60 and the second coil 70 may also be single-thread coils formed by a single wire being wound in a single-thread manner. The first coil 60 and the second coil 70 may also be single-thread strand coils formed by winding in a single-thread manner a strand made of a plurality of wires being stranded. The first coil 60 and the second coil 70 may also be multi-thread strand coils formed by using a plurality of strands made of a plurality of wires being stranded to wind each strand in a multi-thread manner. The types of the first coil 60 and the second coil 70 may be the same or different. The outer and inner diameters of the first coil body 60 and the second coil body 70 can be determined arbitrarily.

The tube 80 is a tubular body having a substantially cylindrical shape. The tube 80 has a distal end portion 81 and a main body part 82. The distal end portion 81 is a tapered-shaped portion which gradually decreases in outer diameter from the proximal end toward the distal end. The main body part 82 is a portion having a substantially constant outer diameter. The tube 80 has an inner cavity with a substantially constant inner diameter. Through the inner cavity of the tube 80, a portion of the proximal end side of the first core shaft 11, a portion of the distal end side of the second core shaft 12, and the wiring 30 are passed. The gap 13 is provided in the inner cavity of the tube 80. The tube 80 improves the rigidity of the medical device with a sensor 1 as the tube 80 is secured to the first core shaft 11 and the second core shaft 12. The tube 80 may be formed using at least any one of nickel-titanium and nickel-titanium alloys, for example.

The distal tip 50 is a member that connects the distal end 111 of the first core shaft 11 to the distal end of the first coil 60. A second joint part 52 is a member that joins the proximal end of the first coil 60, the distal end of the tube 80, and a portion of the first core shaft 11. A third joint part 53 is a member that joins the proximal end of the tube 80, the connector 40, and a portion of the second core shaft 12. The distal tip 50, the second joint part 52, and the third joint part 53 can be formed using any suitable bonding agent. As the bonding agent, at least any one of metal solders such as silver solder, gold solder, zinc, Sn-Ag alloys, and Au-Sn alloys, and adhesives such as epoxy-based adhesives can be used.

Fig. 2 is an enlarged sectional view of the distal end side of the medical device with a sensor 1. The sensor 20 shown in Fig. 2 is positioned at the distal end portion of the medical device with a sensor 1. The sensor 20 is a structure that includes a sensor element for measuring the electrical resistance of bodily fluids such as blood flowing through a blood vessel. Within the sensor 20, the first portion 115 of the first core shaft 11 is passed through. In other words, the first core shaft 11 passes through the sensor 20 in the X-axis direction. The sensor 20 comprises a tubular member 22, a sensor sheet 24, spacers 26, 28, and a first joint part 51.

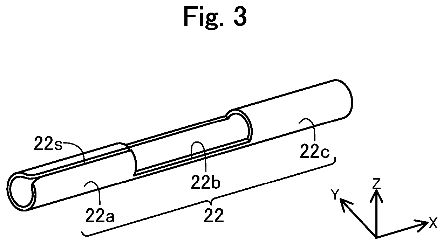

Fig. 3 is an explanatory view illustrating the configuration of the tubular member 22. The tubular member 22 has a proximal end cylindrical portion 22a, a middle connection portion 22b, and a distal end cylindrical portion 22c. The proximal end cylindrical portion 22a is a substantially cylindrical portion located on the proximal end side in the tubular member 22. On the side surface of the proximal end cylindrical portion 22a, a linear slit 22s which passes through the inner and outer sides of the cylinder is formed. The intermediate connecting portion 22b is a portion provided between the proximal end cylindrical portion 22a and the distal end cylindrical portion 22c. The transverse section of the intermediate connecting portion 22b is semicircular. In other words, at the intermediate connection portion 22b, the inner surface of the cylinder is externally exposed. The distal end cylindrical portion 22c is a substantially cylindrical portion located on the distal end side in the tubular member 22. No slit is formed on the side surface of the distal end cylindrical portion 22c. The tubular member 22 can be formed from any resin material.

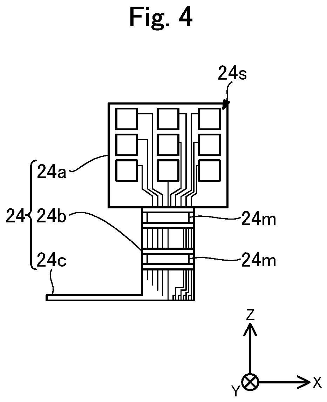

Fig. 4 is an explanatory view illustrating the configuration of the sensor sheet 24. The sensor sheet 24 is a sheet-form member. The sensor sheet 24 has a sensor array 24s, microchips 24m, a large width portion 24a, a small width portion 24b, and sensor wiring 24c.

The sensor array 24s includes nine sensor elements positioned in a grid pattern. The sensor elements are elements that measure the electrical resistance of bodily fluids such as blood flowing through a blood vessel. The microchips 24m transmit signals representing the values measured by the sensor array 24s. The large width portion 24a is a wide, strip-shaped sheet, and the sensor array 24s is positioned on its surface. The small width portion 24b is a strip-shaped sheet that is narrower than the large width portion 24a, and the microchips 24m are positioned on its surface. The sensor wiring 24c is wiring that transmits signals sent by the microchips 24m. The sensor wire 24c is electrically connected to the distal end portion of the wiring 30.

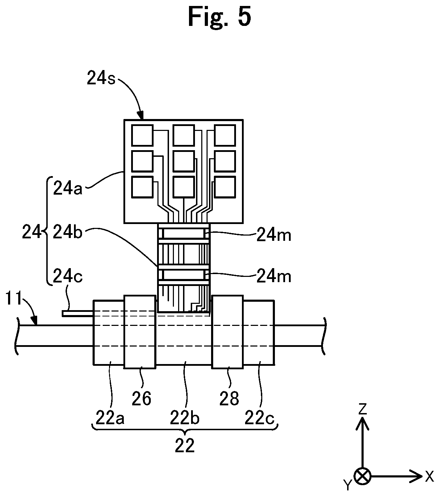

Fig. 5 is an explanatory view explaining a method for making the sensor 20. A worker can make the sensor 20 with procedures as follows. The worker prepares the tubular member 22, the sensor sheet 24, the spacer 26, and the spacer 28. The spacers 26, 28 are annular members having slightly larger inner diameters than the tubular member 22. The spacers 26, 28 may be formed from any resin material. The worker inserts the sensor sheet 24 into the inner side of the tubular member 22 via the slit 22s. Then, as shown in Fig. 5, the sensor sheet 24 is brought about such a state that the large width portion 24a and the small width portion 24b externally protrude from the tubular member 22. The worker inserts the distal end portion of the first core shaft 11 into the inner side of the tubular member 22. The worker fits the annular spacer 26 into the tubular member 22 from the end of the proximal end cylindrical portion 22a side. The worker fits the annular spacer 28 into the tubular member 22 from the end of the distal end cylindrical portion 22c side. Then, as shown in Fig. 5, the tubular member 22 and the sensor sheet 24 are held in the state shown in Fig. 5 by the annular spacers 26, 28. The worker wraps the sensor sheet 24 around the tubular member 22 and the spacers 26, 28. As a result, the small width portion 24b in the sensor sheet 24 is brought into the arrangement that covers the outer periphery of the intermediate connection portion 22b. As shown in Fig. 2, when the sensor sheet 24 is wrapped, one microchip 24m and another microchip 24m on the small width portion 24b are positioned around the tubular member 22 with those being stacked one on top of the other. The large width portion 24a in the small width portion 24b is wrapped around the outer periphery of the spacers 26, 28. As a result, nine sensor elements of the sensor array 24s are positioned at the outermost periphery of the sensor 20. In other words, three sensor elements are positioned at 120° intervals along the circumferential direction.

Returning to Fig. 2, description is continued. The first joint part 51 is a member that joins the sensor 20, the first core shaft 11, the first coil 60, and the second coil 70. The first joint part 51 is positioned within the tubular member 22 of the sensor 20 and joins the sensor 20 to the first core shaft 11. The first joint part 51 is positioned at the outer peripheral portion on the more distal end side than the spacer 28 of the sensor 20, and joins the sensor 20 to the first coil 60. The first joint part 51 is positioned at the outer peripheral portion on the more proximal end side than the spacer 26 of the sensor 20, and joins the sensor 20 to the second coil 70. The first joint part 51 can be formed by any bonding agent. As the bonding agent, at least any one of metal solders such as silver solder, gold solder, zinc, Sn-Ag alloys, and Au-Sn alloys, and adhesives such as epoxy-based adhesives can be used. As shown in Fig. 2, the outer diameter Φ60 of the first coil 60 is equal to the outer diameter Φ70 of the second coil 70. The outer diameter Φ20 of the sensor 20 is smaller than the outer diameter Φ60 of the first coil 60. The outer diameter Φ20 of the sensor 20 is smaller than the outer diameter Φ70 of the second coil 70.

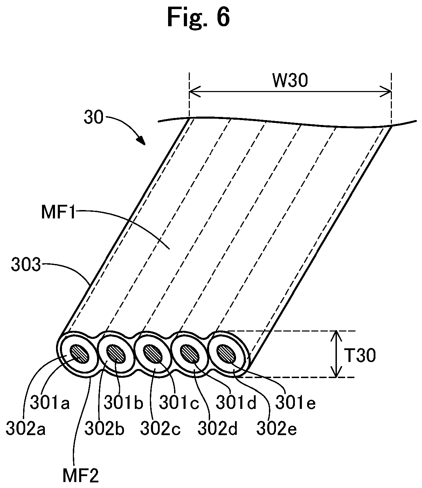

Fig. 6 is an explanatory view illustrating the configuration of the wiring 30. The wiring 30 is a connection line that electrically connects the sensor 20 to the connector 40. As shown in Fig. 1, the distal end portion 31 of the wiring 30 is electrically connected to the sensor 20. The proximal end portion 36 of the wiring 30 is electrically connected to the connector 40. The wiring 30 transmits signals from the sensor 20 to an external device via the connector 40. As shown in Fig. 6, the wiring 30 comprises leads 301a-301e, inner coating portions 302a-302e, and an outer coating portion 303.

The leads 301a-301e are conductive metal conductors. The inner coating portions 302a-302e are tubes formed from insulating resins. The inner coating portion 302a covers the outer peripheral surface of the lead 301a. Similarly, the inner coating portions 302b-302e cover the outer peripheral surfaces of the leads 301b-301e, respectively. The inner coating portions 302a-302e can be formed from insulating resins, for example, polyimide resins. The five leads 301a-301e are positioned horizontally in a single row with each being covered by the inner coating portions 302a-302e. The outer coating portion 303 covers and integrally holds outer peripheral surfaces of the leads 301a-301e and the inner coating portions 302a-302e. The outer coating portion 303 can be formed from insulating resins, for example, urethane resins.

In the wiring 30, the direction in which the leads 301a-301e and the inner coating portions 302a-302e are lined is also referred to as the "width direction." In the wiring 30, the direction perpendicular to both the length and width directions is also referred to as the "thickness direction." As shown in Fig. 6, the wiring 30 has a length T30 in the thickness direction which is smaller than a length W30 in the width direction. The wiring 30 has a flat shape with a transverse sectional shape in which the thickness direction is smaller than the width direction. The transverse section of the wiring 30 is non-circular. Of the side surfaces of the wiring 30, as for a pair of main surfaces having relatively large areas, one is also referred to as a "first main surface MF1" and the other as a "second main surface MF2." The first main surface MF1 and the second main surface MF2 are opposed to each other.

Returning to Fig. 1, description is continued. The distal end portion 31 of the wiring 30 is electrically connected to the sensor wiring 24c of the sensor 20 with the inner coating portions 302a-302e and the outer coating portion 303 being removed. The distal end portion 31 of the wiring 30 is straight. "Electrically connected" means that the connection is provided in a state where current can flow; in other words, it means that the connection is provided in a state where electricity can be conducted.

The wiring 30 is wound spirally around the outer peripheral surface of the first core shaft 11 in a first section P1. The first section P1 is a section located on the more proximal end side than than the sensor lead 24c of the sensor 20, and on the more distal end side than the second joint part 52. The wiring 30 in the first section P1 has a first roll portion 32, a second roll portion 33, and a third roll portion 34. In the first roll portion 32, the wiring 30 is loosely wound around the second portion 116 of the first core shaft 11. In the first roll portion 32, the adjacent spacings of the wiring 30 gradually decrease from the distal end side toward the proximal end side. In the second roll portion 33, the wiring 30 is closely wound around the third portion 117 and the fourth portion 118 of the first core shaft 11. In the third roll portion 34, the wiring 30 is loosely wound around the fourth portion 118 of the first core shaft 11. In the third roll portion 34, the adjacent spacings of the wiring 30 gradually increase from the distal end side toward the proximal end side. In other words, in the first roll portion 32 and the third roll portion 34, the adjacent spacings of the wiring 30 gradually decrease as they approach the second roll portion 33. By virtue of the wiring 30 being wound around the first core shaft 11, the center axis of the first core shaft 11 can be maintained in alignment with the center axis O of the medical device with a sensor 1. As a result, when the medical device with a sensor 1 is in use, stress concentration in a portion of the first core shaft 11 can be suppressed.

In the second section P2, the wiring 30 extends straight along the outer peripheral surfaces of the first core shaft 11 and the second core shaft 12. In other words, in the second section P2, the wiring 30 is not helical. The second section P2 is a section which is located on the more proximal end side than the second joint part 52 and extends to the inner side of the connector 40. The wiring 30 in the second section P2 has a straight portion 35 and a proximal end portion 36. In the straight portion 35, the wiring 30 extends straight along the fourth portion 118 of the first core shaft 11 and the fifth portion 125 of the second core shaft 12. Also in the gap 13, the wiring 30 extends along the long axis direction of the medical device with a sensor 1. At the proximal end portion 36, the wiring 30 is inserted into the inner side of the connector 40 and is electrically connected to the connector 40.

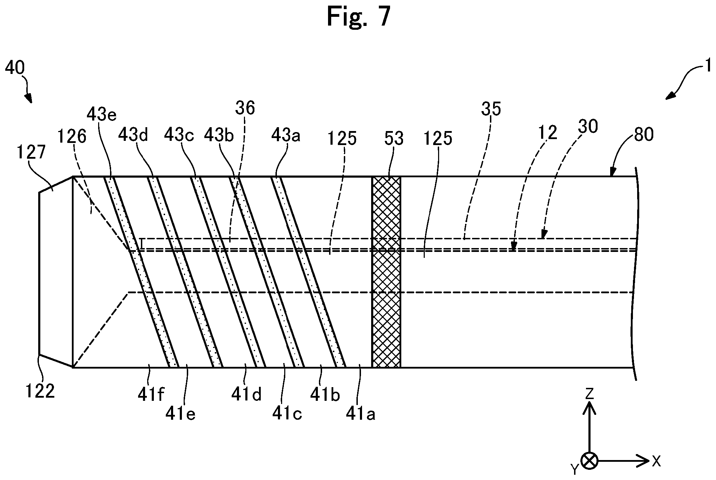

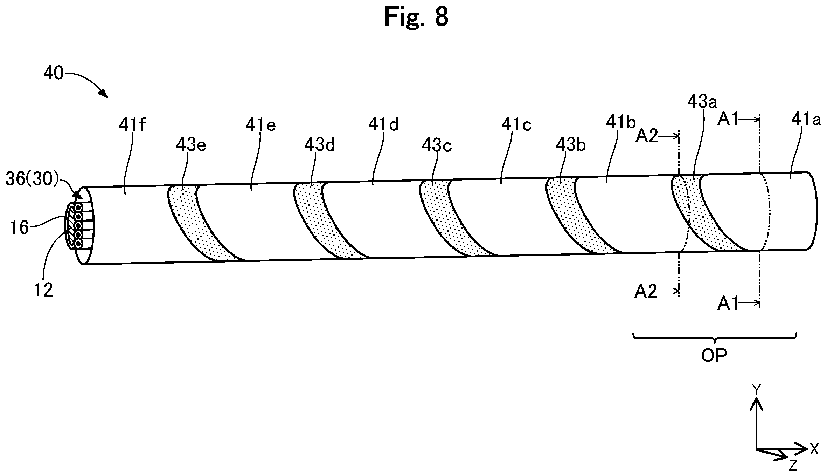

Fig. 7 is an enlarged view of the proximal end side in the medical device with a sensor 1. In Fig. 7, while the appearance of the medical device with a sensor 1 is represented in solid lines, the second core shaft 12 and the wiring 30 which are positioned within the inner cavities of the connector 40 and tube 80 are represented in dashed lines. Fig. 8 is an explanatory view illustrating the configuration of the connector 40. The connector 40 is positioned at the proximal end portion of the medical device with a sensor 1. The connector 40 is a terminal that is connected to an external device that processes output signals from the sensor 20. The second core shaft 12 and the wiring 30 are passed through the inner side of the connector 40. The connector 40 is joined to the proximal end of the tube 80 by the third joint part 53. As shown in Fig. 8, the connector 40 comprises six tubes 41a-41f and five insulators 43a-43e.

The tubes 41a-41f are conductive hollow electrodes. In the present embodiment, tubes 41a-41f are cylindrical. The tubes 41a-41f can be formed from conductive metals, for example, platinum-iridium alloys. The first tube 41a is located on the most distal end side in the connector 40 and on the more proximal end side than the sensor 20. The distal end face of the first tube 41a is vertical to the X-axis direction. The proximal end face of the first tube 41a is inclined with respect to the X-axis direction. The second tube 41b is located on the more proximal end side than the first tube 41a. The distal end face of the second tube 41b is inclined with respect to the X-axis direction. The proximal end face of the second tube 41b is inclined with respect to the X-axis direction. The orientation of tilt and the angle of tilt in the distal end face and the proximal end face of the second tube 41b are the same. The third tube 41c is located on the more proximal end side than the second tube 41b. The fourth tube 41d is located on the more proximal end side than the third tube 41c. The fifth tube 41e is located on the more proximal end side than the fourth tube 41d. The third tube 41c, the fourth tube 41d, and the fifth tube 41e have the same configurations as that of the second tube 41b. The sixth tube 41f is located on the most proximal end side in the connector 40 and on the more proximal end side than the fifth tube 41e. The distal end face of the sixth tube 41f is inclined with respect to the X-axis direction. The proximal end face of the sixth tube 41f is vertical with respect to the X-axis direction.

The insulators 43a-43e are insulating members that insulate adjacent tubes 41a-41f from one another. The insulators 43a-43e can be formed from insulating adhesives, for example, polyimide resins. The first insulator 43a is positioned and sandwiched between the first tube 41a and the second tube 41b. As shown, a portion of the first insulator 43a is externally exposed. "Externally exposed" means that it is visible in appearance. The second insulator 43b is positioned and sandwiched between the second tube 41b and the third tube 41c. A portion of the second conductor 42b is externally exposed. The third insulator 43c is positioned and sandwiched between the third tube 41c and the fourth tube 41d. A portion of the third insulator 43c is externally exposed. The fourth insulator 43d is positioned and sandwiched between the fourth tube 41d and the fifth tube 41e. A portion of the fourth insulator 43d is externally exposed. The fifth insulator 43e is positioned and sandwiched between the fifth tube 41e and the sixth tube 41f. A portion of the fifth insulator 43e is externally exposed.

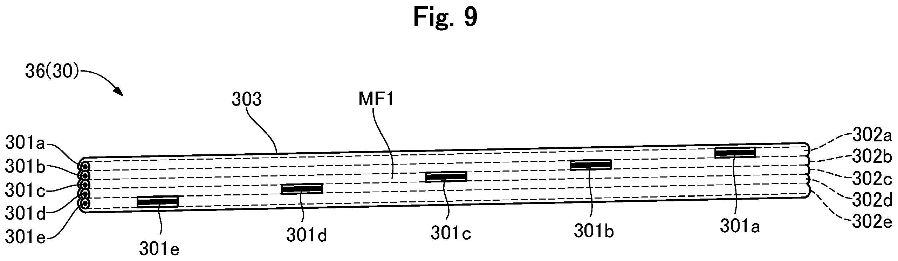

Fig. 9 is an explanatory view illustrating the configuration of the proximal end portion 36 of the wiring 30. As shown in Fig. 7, the proximal end portion 36 of the wiring 30 is positioned within the first to fifth tubes 41a-41e in the connector 40. As for the wiring 30, the inner coating portion 302a and the outer coating portion 303 have been partly peeled off to expose the lead 301a in a position corresponding to the first tube 41a, as shown in Fig. 9. The exposed lead 301a is electrically connected to the first tube 41a. For the wiring 30, the inner coating portion 302b and the outer coating portion 303 have been partly peeled off to expose the lead 301b in a position corresponding to the second tube 41b. The exposed lead 301b is electrically connected to the second tube 41b. For the wiring 30, the inner coating portion 302c and the outer coating portion 303 have been partly peeled off to expose the lead 301c in a position corresponding to the third tube 41c. The exposed lead 301c is electrically connected to the third tube 41c. For the wiring 30, the inner coating portion 302d and the outer coating portion 303 have been partly peeled off to expose the lead 301d in a position corresponding to the fourth tube 41d. The exposed wiring 301d is electrically connected to the fourth tube 41d. For the wiring 30, the inner coating portion 302e and the outer coating portion 303 have been partly peeled off to expose the lead 301e in a position corresponding to the fifth tube 41e. The exposed wiring 301e is electrically connected to the fifth tube 41e. In Fig. 7, the fifth portion 125 of the second core shaft 12 is positioned within the first to fifth tubes 41a-41e in the connector 40. In the connector 40, the sixth portion 126 of the second core shaft 12 is positioned within the sixth tube 41f. The seventh portion 127 of the second core shaft 12 protrudes externally from the proximal end of the connector 40.

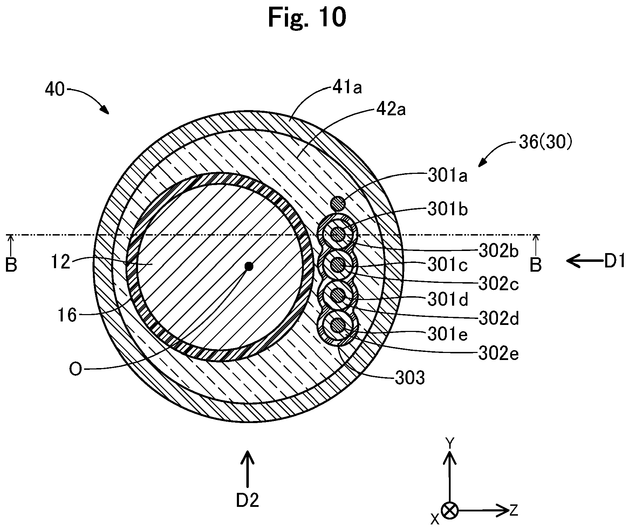

Fig. 10 is a transverse sectional view of the connector 40 along the line A1-A1 of Fig. 8. In the connector 40, the second core shaft 12 and the wiring 30 are positioned within the first tube 41a. The outer peripheral surface of the second core shaft 12 is coated by a second insulating tube 16 which has insulation properties. For the wiring 30, the inner coating portion 302a and the outer coating portion 303 have been partly peeled off to expose the lead 301a. A conductive first conductor 42a is filled within the first tube 41a. The first conductor 42a electrically connects the first tube 41a to the wiring 301a, and secures the second core shaft 12 and the wiring 30 which are positioned within the first tube 41a. The first conductor 42a can be formed with a conductive adhesive containing metal powder, for example. The first conductor 42a may be formed with a conductive solder paste.

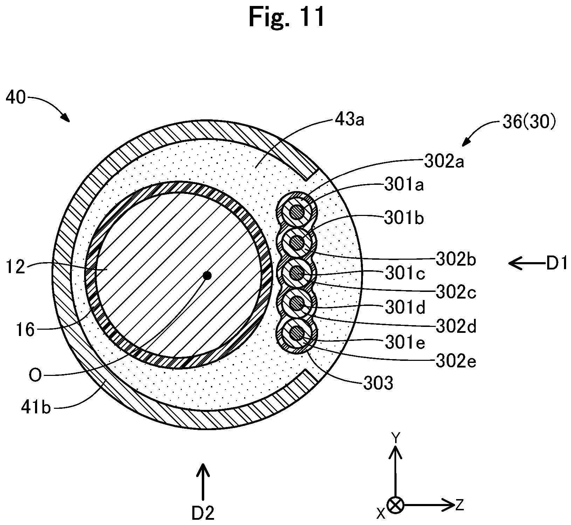

Fig. 11 is a transverse sectional view of the connector 40 along the line A2-A2 of Fig. 8. In the connector 40, at a position where the first insulator 43a is provided, the second core shaft 12 and the wiring 30 are positioned within the second tube 41b. The outer peripheral surface of the second core shaft 12 is coated by a second insulating tube 16 which has insulation properties. In the wiring 30, there are no exposed portions for the conductors 301a-301e. The second tube 41b is filled with the insulating first insulator 43a. The first insulator 43a electrically insulates the second tube 41b from the first tube 41a, and secures the second core shaft 12 and the wiring 30 which are positioned within the second tube 41b. The distal end face of the second tube 41b is inclined with respect to the X-axis direction. For this reason, in a transverse section including the first insulator 43a, the second tube 41b has a circular shape which is partly cut-out in the circumferential direction. The cut-out portion in the second tube 41b is filled with the first insulator 43a. The first insulator 43a which is filled into the cut-out portion of the second tube 41b is externally exposed.

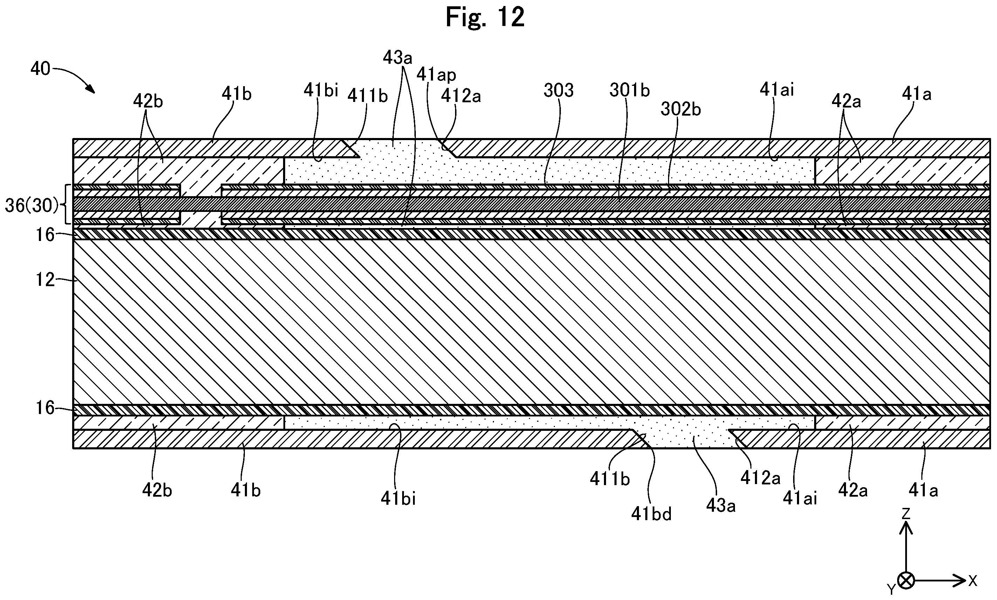

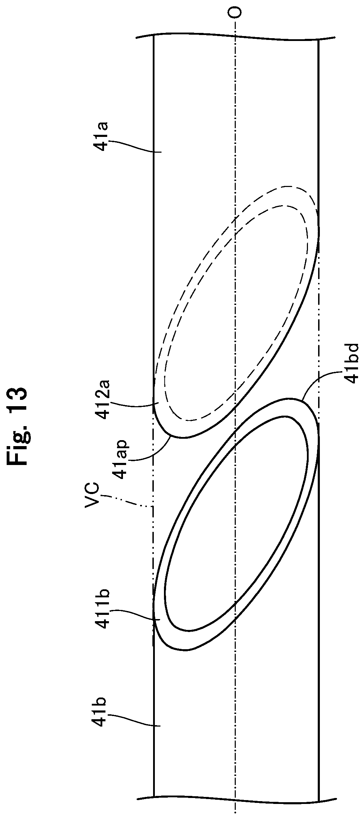

Fig. 12 is a longitudinal sectional view of the connector 40 along the line B-B of Fig. 10. Fig. 13 is a view illustrating the relationship between the first tube 41a and the second tube 41b. In Fig. 13, the first insulator 43a is not shown for the sake of explanation. Fig. 13 illustrates a virtual cylindrical surface VC by two-dot chain lines in which the outer peripheral surface of the first tube 41a is extended to the proximal end side. The virtual cylindrical surface VC coincides with the outer peripheral surface of the second tube 41b. "Coincide" includes "roughly coincide" and allows minor discrepancies resulting from manufacturing tolerances or the like. In other words, the first tube 41a and the second tube 41b are positioned such that their outer peripheral surfaces coincide with the virtual cylindrical surface VC as if they were a single tube. The first tube 41a has a double-elliptical surface 412a at the proximal end located on the second tube 41b side. The double-elliptical surface is formed by the outer and inner peripheries of the tube. The double-elliptical surface is the cross-section of the tube wall. As shown in Fig. 13, the double-elliptical surface 412a is the proximal end side end face of the first tube 41a. The entire double-elliptical surface 412a is inclined with respect to the long axis of the medical device with a sensor 1. The second tube 41b has a double-elliptical surface 411b at the distal end located on the first tube 41a side. As shown in Fig. 13, the double elliptical surface 411b is the distal end side end face of the second tube 41b. The entire double elliptical surface 411b is inclined with respect to the long axis of the medical device with a sensor 1. The double elliptical surface 412a of the first tube 41a and the double elliptical surface 411b of the second tube 41b are parallel. In other words, the double elliptical surface 412a of the first tube 41a and the double elliptical surface 411b of the second tube 41b are inclined in the same direction and at the same angle. A proximal end 41ap of the first tube 41a is located on the proximal end side than a distal end 41bd of the second tube 41b.

As shown in Fig. 12, the first insulator 43a is positioned between the double elliptical surface 412a and the double elliptical surface 411b. In other words, the first tube 41a is joined to the first insulator 43a at the double elliptical surface 412a. The second tube 41b is joined to the first insulator 43a at the double elliptical surface 411b. The double elliptical surface 412a and the double elliptical surface 411b are parallel. Therefore, the joint surface between the first tube 41a and the first insulator 43a is parallel to the joint surface between the second tube 41b and the first insulator 43a. At the proximal end portion of the first tube 41a, the first insulator 43a is further positioned on an inner peripheral surface 41ai of the first tube 41a. At the distal end portion of the second tube 41b, the first insulator 43a is further positioned on an inner peripheral surface 41bi of the second tube 41b. The second core shaft 12 passes through the first insulator 43a. The wiring 30 passes through the first insulator 43a.

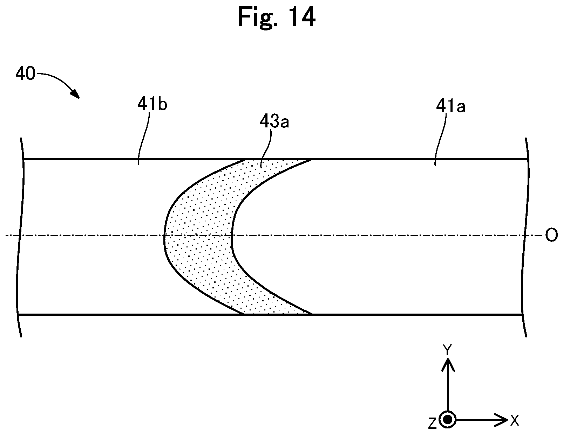

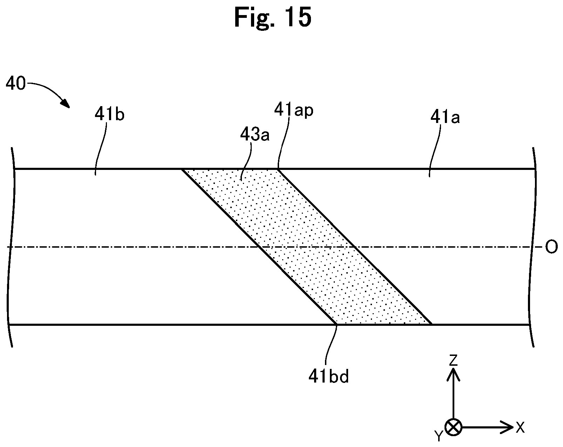

Fig. 14 is an external view of the connector 40 when viewed from a first radial direction D1. Fig. 15 is an external view of the connector 40 when viewed from a second radial direction D2. As shown in Fig. 10, the first radial direction D1 is a direction from the outer side of the medical device with a sensor 1 toward the center O along the Z-axis direction. The second radial direction D2 is a direction from the outer side of the medical device with a sensor 1 toward the center O along the Y-axis. The first radial direction D1 and the second main surface MF2 shown in Fig. 10 are examples only. The first radial direction D1 and the second radial direction D2 may be determined optionally, provided they differ from one another. As shown in Fig. 14, the shape of the exposed portion of the first insulator 43a when viewed from the first radial direction D1 is approximately C-shaped. The shape of the exposed portion of the first insulator 43a when viewed from a certain radial direction is a two-dimensional shape obtained by projecting the outline of a portion of the exposed portion of the first insulator 43a that is visible from that radial direction onto a plane perpendicular to that radial direction. As shown in Fig. 15, the shape of the exposed portion of the first insulator 43a when viewed from the second radial direction D2 has a parallelogram shape. Thus, the shape of the exposed portion of the first insulator 43a when viewed from the first radial direction D1 is different from the shape of the exposed portion of the first insulator 43a when viewed from the second radial direction D2. Figs. 14 and 15 have illustrated the shapes of the exposed portion of the first insulator 43a when viewed from the first radial direction D1 and when viewed from the second radial direction D2 illustrated in Fig. 10. The exposed portion of the first insulator 43a has different shapes not only when viewed from the first and second radial directions D1 and D2 illustrated in Fig. 10, but also when viewed from different radial directions from each other.

Figs. 10-15 have described the first tube 41a and the second tube 41b joined by the first insulator 43a. The second tube 41b and the third tube 41c which are joined by the second insulator 43b also have the similar configuration. The third tube 41c and the fourth tube 41d which are joined by the third insulator 43b also have the similar configuration. The fourth tube 41d and the fifth tube 41e which are joined by the fourth insulator 43d also have the similar configuration. As for the fifth tube 41e and the sixth tube 41f which are joined by the fifth insulator 43e, they have the similar configurations, except that the wiring 30 is not positioned within the sixth tube 41f but the sixth portion 126 of the second core shaft 12 is arranged there. The sixth portion 126 and the seventh portion 127 of the second core shaft 12 are not coated by the second insulating tube 16. For this reason, in the inner side of the sixth tube 41f, the sixth tube 41f and the second core shaft 12 are electrically connected by a conductor positioned within the sixth tube 41f.

The shape of the exposed portion of the second insulator 43b when viewed from the first radial direction D1 is different from the shape of the exposed portion of the second insulator 43b when viewed from the second radial direction D2. The shape of the exposed portion of the third insulator 43c when viewed from the first radial direction D1 is different from the shape of the exposed portion of the third insulator 43c when viewed from the second radial direction D2. The shape of the exposed portion of the fourth insulator 43d when viewed from the first radial direction D1 is different from the shape of the exposed portion of the fourth insulator 43d when viewed from the second radial direction D2. The shape of the exposed portion of the fifth insulator 43e when viewed from the first radial direction D1 is different from the shape of the exposed portion of the fifth insulator 43e when viewed from the second radial direction D2. As shown in Fig. 8, the shapes of the exposed portions when viewed from the first radial direction D1 are identical as for all the first to fifth insulators 43a-43e. The shapes of the exposed portions when viewed from the second radial direction D2 are identical as for all the first to fifth insulators 43a-43e.

When an operator pushes the connector 40 into the terminal receptacle of an external device in order to connect the connector 40 to the external device, force in the X-axis direction acts on the connector 40, causing the connector 40 to be flexed. In the connector 40, the resin insulators 43a-43e have lower rigidity than the metal tubes 41a-41f. For this reason, when connector 40 is connected to the external device, bending stress tends to act on the relatively less rigid insulators 43a-43e. According to the medical device with a sensor 1 of the first embodiment, the insulators 43a-43e have the shapes of the exposed portions when viewed from the first radial direction D1 which are different from the shapes of the exposed portions when viewed from the second radial direction D2 which is different from the first radial direction D1. In other words, the insulators 43a-43e do not have right cylindrical shapes. Therefore, even if bending stress acts in the vicinity of the insulators 43a-43e when the connector 40 is connected to the external device, stress is less likely to concentrate in the insulators 43a-43e. As a result, according to the medical device with a sensor 1, the breakage of the insulators 43a-43e can be inhibited.

According to the medical device with a sensor 1 of the first embodiment, since the insulators 43a-43e are formed with insulating adhesives, the connector 40 which includes the first tube 41a, the second tube 41b, and the first insulator 43a can be easily manufactured. According to the medical device with a sensor 1 of the first embodiment, it comprises the second core shaft 12 that passes through the insulators 43a-43e. Therefore, the strength of a portion where the insulators 43a-43e are positioned can be improved in the connector 40. As a result, the breakage of the insulators 43a-43e can be further inhibited to improve safety of procedures.

According to the medical device with a sensor 1 of the first embodiment, the proximal end 41ap of the first tube 41a is located on the more proximal end side than the distal end 41bd of the second tube 41b. Therefore, stress is distributed more evenly across the first insulator 43a, thereby preventing damage to the first insulator 43a more effectively. According to the medical device with a sensor 1 of the first embodiment, the virtual cylindrical surface VC in which the outer peripheral surface of the first tube 41a is extended to the proximal end side substantially coincides with the outer peripheral surface of the second tube 41b. For this reason, the first tube 41a and the second tube 41b can be configured to be like a single tube.

According to the medical device with a sensor 1 of the first embodiment, at least any one of the double elliptical surface 412a of the first tube 41a and the double elliptical surface 411b of the second tube 41b includes a portion that is inclined with respect to the long axis of the medical device with a sensor 1. Consequently, even if bending stress acts in the vicinity of the first insulator 43a when the connector 40 is connected to an external device, stress on the first insulator 43a is distributed more evenly, thereby preventing damage to the first insulator 43a more effectively. The same applies to the insulators 43b-43e.

According to the medical device with a sensor 1 of the first embodiment, at least a portion of the joint surface between the first tube 41a and the first insulator 43a is substantially parallel to at least a portion of the joint surface between the second tube 41b and the first insulator 43a. As a result, a portion consisting solely of the first tube 41a disappears in the longitudinal direction of the medical device with a sensor 1. In other words, the metal tubes 41a, 41b are at least partly included in the transverse section of any portion along the longitudinal direction of the medical device with a sensor 1. Therefore, stress is distributed more evenly across the first insulator 43a, thereby preventing damage to the first insulator 43a more effectively. The same applies to the insulators 43b-43e.

According to the medical device with a sensor 1 of the first embodiment, at least any one of the first tube 41a and the second tube 41b is joined to the first insulator 43a at the inner peripheral surfaces 41ai, 41bi. Therefore, since the joint strength between the first and second tubes 41a, 41b and the first insulator 43a can be improved, the connector 40 less prone to break can be provided. The same applies to the insulators 43b-43e.

According to the medical device with a sensor 1 of the first embodiment, it comprises the second insulator 43b that is sandwiched between the second tube 41b and the third tube 41c, a portion of which is externally exposed, and the shape of the exposed portion of which when viewed from the first radial direction D1 is different from the shape of the exposed portion of which when viewed from the second radial direction D2. For this reason, the breakage of the second insulator 43b can be inhibited. According to the medical device with a sensor 1 of the first embodiment, the shape of the exposed portion of the first insulator 43a is substantially the same as the shape of the exposed portion of the second insulator 43b when viewed from the first radial direction D1. Therefore, it is possible to easily manufacture the medical device with a sensor 1 which can inhibit the breakage of the insulators 43a-43e.

### <Second Embodiment>

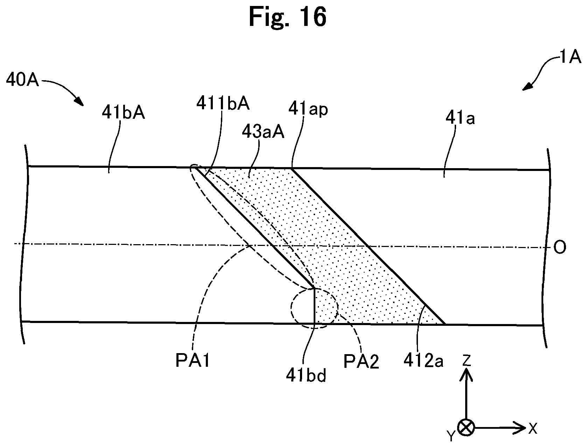

Fig. 16 is an external view of a connector 40A of a second embodiment when viewed from the second radial direction D2. A medical device with a sensor 1A of the second embodiment comprises the connector 40A instead of the connector 40 in the configuration described in the first embodiment. The connector 40A comprises a second tube 41bA instead of the second tube 41b, and a first insulator 43aA instead of the first insulator 43a in the configuration described in the first embodiment.

The second tube 41bA has a double elliptical surface 411bA at the distal end located on the first tube 41a side. A first portion PA1 of the double elliptical surface 411bA is inclined with respect to the long axis direction of the medical device with a sensor 1A. A second portion PA2 of the double elliptical surface 411bA is vertical to and is not inclined with respect to the long axis direction of the medical device with a sensor 1A. For this reason, the double elliptical surface 412a and the double elliptical surface 411bA are parallel at the first portion PA1 of the double elliptical surface 411bA. The double elliptical surface 412a and the double elliptical surface 411bA are not parallel at the second portion PA2 of the double elliptical surface 411bA. The first insulator 43aA is positioned between the double elliptical surface 412a and the double elliptical surface 411bA, and joins the first tube 41a to the second tube 41bA.

As such, the configuration of the connector 40A can be modified in various ways. The double elliptical surface 411bA of the second tube 41bA may include the second portion PA2 that is not inclined with respect to the long axis direction of the medical device with a sensor 1A. The position and the size of the second portion PA2 may be determined optionally. Also for the first tube 41a, the third to sixth tubes 41c-41f, they may be configured to include the first section PA1 and the second section PA2. The medical device with a sensor 1A of the second embodiment as described above can also achieve the same effects as those of the first embodiment.

### <Third Embodiment>

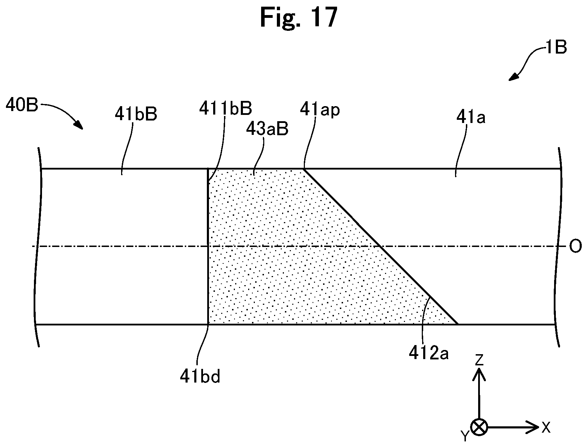

Fig. 17 is an external view of a connector 40B of a third embodiment when viewed from the second radial direction D2. A medical device with a sensor 1B of the third embodiment comprises the connector 40B instead of the connector 40 in the configuration described in the first embodiment. The connector 40B comprises a second tube 41bB instead of the second tube 41b, and a first insulator 43aB instead of the first insulator 43a in the configuration described in the first embodiment.

The second tube 41bB has a double circular surface 411bB at the distal end located on the first tube 41a side. The double circular surface is formed by the outer and inner peripheries of the tube. The entire double circular surface 411bB is vertical to the long axis direction of the medical device with a sensor 1B and is not inclined thereto. For this reason, the double elliptical surface 412a and the double circular surface 411bB are not parallel. The joint surface between the first tube 41a and the first insulator 43aB on the double elliptical surface 412a is not parallel to the joint surface between the second tube 41bB and the first insulator 43aB on the double circular surface 411bB. The proximal end 41ap of the first tube 41a is located on the more distal end side than the distal end 41bd of the second tube 41bB. The first insulator 43aB is positioned between the double elliptical surface 412a and the double circular surface 411bB, and joins the first tube 41a to the second tube 41bB. The first insulator 43aB has a trapezoidal shape when viewed from the second radial direction D2.

Thus, the configuration of the connector 40B can be modified in various ways. The end face of the second tube 41bB may be the double circular surface 411bB that is not inclined to the long axis direction of the medical device with a sensor 1B. Also for the first tube 41a, the third to sixth tubes 41c-41f, a part of the end faces may be a double circular surface that is not inclined to the long axis direction of the medical device with a sensor 1B. The medical device with a sensor 1B of the third embodiment as described above can also achieve the same effects as those of the first embodiment.

### <Fourth Embodiment>

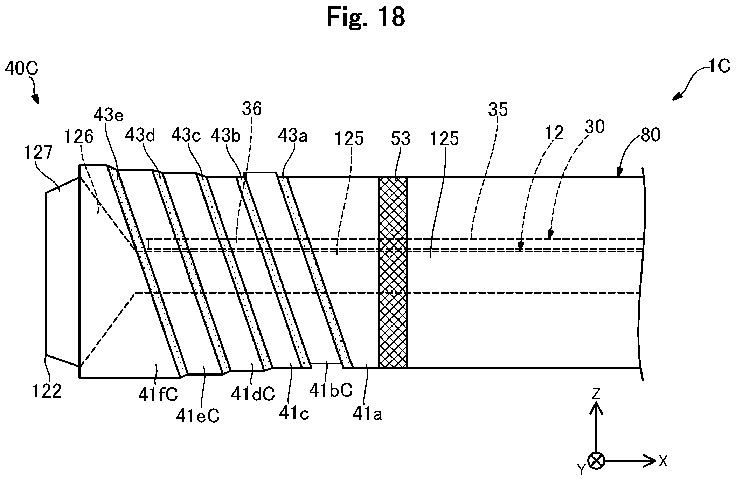

Fig. 18 is an enlarged view of the proximal end side of a medical device with a sensor 1C of a fourth embodiment. The medical device with a sensor 1C of the fourth embodiment comprises a connector 40C instead of the connector 40 in the configuration described in the first embodiment. The connector 40C comprises a second tube 41bC instead of the second tube 41b, a fourth tube 41dC instead the fourth tube 41d, a fifth tube 41eC instead of the fifth tube 41e, and a sixth tube 41fC instead of the sixth tube 41f.

The second tube 41bC has the same outer diameter as the first tube 41a. The second tube 41bC is positioned at a location offset with respect to the first tube 41a in the circumferential direction. The imaginary cylindrical surface in which the outer peripheral surface of the first tube 41a is extended to the proximal end side does not coincide with the outer peripheral surface of the second tube 41bC. The fourth tube 41dC has a larger outer diameter than the third tube 41c. The fifth tube 41eC has a larger outer diameter than the fourth tube 41dC. The sixth tube 41fC has a larger outer diameter than the fifth tube 41eC. The imaginary cylindrical surface in which the outer peripheral surface of the first tube 41a is extended to the proximal end side does not coincide with the outer peripheral surface of the fourth tube 41dC. The imaginary cylindrical surface in which the outer peripheral surface of the first tube 41a is extended to the proximal end side does not coincide with the outer peripheral surface of the fifth tube 41eC. The imaginary cylindrical surface in which the outer peripheral surface of the first tube 41a is extended to the proximal end side does not coincide with the outer peripheral surface of the sixth tube 41fC.

Thus, the configuration of the connector 40C can be modified in various ways. The imaginary cylindrical surface in which the outer peripheral surface of the first tube 41a is extended to the proximal end side may not coincide with the outer peripheral surface of the second tube 41bC. As shown in Fig. 18, at least a part of the tubes 41a-41f may gradually increase in outer diameter from the distal end toward the proximal end. As opposed to Fig. 18, at least a part of the tubes 41a-41f may gradually decrease in outer diameter from the distal end toward the proximal end. The medical device with a sensor 1C of the fourth embodiment as described above can also achieve the same effects as those of the first embodiment.

### <Fifth Embodiment>

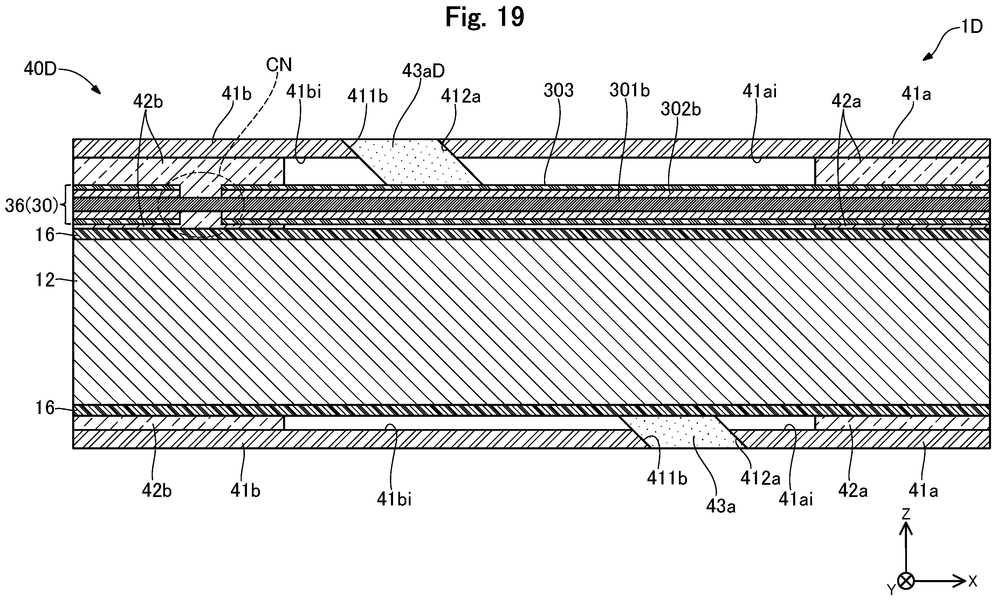

Fig. 19 is a longitudinal sectional view of a connector 40D of a fifth embodiment. A medical device with a sensor 1D of the fifth embodiment comprises the connector 40D instead of the connector 40 in the configuration described in the first embodiment. The connector 40D comprises a first insulator 43aD instead of the first insulator 43a in the configuration described in the first embodiment. The first insulator 43aD is positioned only between the opposing first tube 41a and second tube 41b, and is not positioned on the inner peripheral surface 41ai of the first tube 41a and the inner peripheral surface 41bi of the second tube 41b. In the illustrated example, a portion where the first conductor 42a is not positioned is hollow in the inner peripheral surface 41ai of the first tube 41a. A portion where the second conductor 42b is not positioned is hollow in the inner peripheral surface 41bi of the second tube 41b.

In this way, the configuration of the connector 40D can be modified in various ways. In the connector 40D, the first insulator 43aD may not be positioned on the inner peripheral surface 41ai of the first tube 41a and on the inner peripheral surface 41bi of the second tube 41b. Also for the second to fifth insulators 43b-43e, they may be positioned only between adjacent tubes and may not be positioned on the inner peripheral surface of the tube. The medical device with a sensor 1D of the fifth embodiment as described above can also achieve the same effects as those of the first embodiment.

### <Sixth Embodiment>

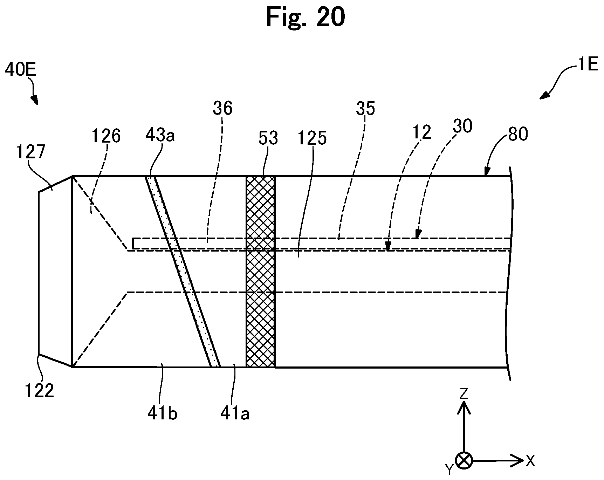

Fig. 20 is an enlarged view of the proximal end side of a medical device with a sensor 1E of a sixth embodiment. The medical device with a sensor 1E of the sixth embodiment comprises a connector 40E instead of the connector 40 in the configuration described in the first embodiment. The connector 40E does not comprise the third to sixth tubes 41c-41f and the second to fifth insulators 43b-43e described in the first embodiment. The connector 40E comprises the first tube 41a, the second tube 41b, the first insulator 43a, the first conductor 42a, and a second conductor 42b only.

In this manner, the configuration of the connector 40E can be modified in various ways. The number of conductive tubes included in the connector 40E may be two. The number of conductive tubes included in the connector 40E may be any number of two or more; it may be three or ten. The number of insulators included in the connector 40E is equal to the number of conductive tubes minus one. The medical device with a sensor 1E of the sixth embodiment as described above can also achieve the same effects as those of the first embodiment.

### <Modified Examples of Present Embodiments>

The present disclosure is not limited to the above embodiments, and can be implemented in various aspects without departing from the gist thereof. For example, the following modified examples are also possible.

### [Modified Example 1]

The configurations of the medical devices with a sensor 1, 1A-1E have been illustrated in the above first to sixth embodiments. However, the configuration of the medical device with a sensor can be modified in various ways. For example, the sensor 20 is not limited to a sensor that measures the electrical resistance of bodily fluids such as blood flowing within a blood vessel. The sensor 20 may be a sensor that measures blood pressure, i.e., intravascular pressure. The measured blood pressure can be used to derive coronary flow reserve. Coronary flow reserve is also referred to as fractional flow reserve, or FFR for short. Coronary flow reserve is pressure at the proximal end of stenosis versus pressure at the distal end of stenosis, and can be used as an indicator of severity of physiological stenosis. For example, the sensor 20 may not be formed using the sheet-form sensor sheet 24. The sensor 20 may be configured to secure any number of sensor elements to the outer peripheral surface of the tubular member 22 in any manner.

For example, in the medical device equipped with a sensor, at least a portion of the first coil 60, the second coil 70, and the tube 80 may be covered with resin coatings. In the medical device with a sensor, the entire body except the connector 40 may be covered with resin coatings. As coating resins, any one of hydrophilic resins and hydrophobic resins can be used.

For example, the first core shaft 11 and the second core shaft 12 may be positioned without the gap 13 being made therebetween. In other words, the proximal end face of the first core shaft 11 may come into contact with the proximal end face of the second core shaft 12. For example, a single core shaft may be used instead of the first core shaft 11 and the second core shaft 12. In this case, the tube 80 may be omitted.

For example, an inner coil body may be provided within the first coil 60. For example, a tube may be used instead of the second coil 70. As the tube, a flexible resin tube, or a metal tube with a slit being provided may used. For example, a coil may be used instead of the tube 80.

For example, the leads may not be tied together in the wiring 30. In other words, the outer coating portion 303 of the wiring 30 may be omitted. In the wiring 30, the leads may be tied using a tying member other than the outer coating portion 303. In the wiring 30, a portion of the leads may be tied, and the remaining portion may not be tied. The number of the leads 301a-301e included in the wiring 30 may not be five. The wiring 30 may have the length T30 in the thickness direction which is not smaller than the length W30 in the width direction. The wiring 30 may have the length T30 in the thickness direction which is the same as the length W30 in the width direction.

### [Modified Example 2]

The above first to sixth embodiments have illustrated the configurations of the connectors 40, 40A-40E. However, the configuration of the connector can be modified in various ways. For example, the shape of the exposed portion of the first insulator 43a when viewed from the first radial direction D1 shown in Fig. 14 and the shape of the exposed portion of the first insulator 43a when viewed from the second radial direction D2 shown in Fig. 15 are merely examples. As for the first insulator 43a, the shape of the exposed portion when viewed from the first radial direction D1 and the shape of the exposed portion when viewed from the second radial direction D2 may be of any shape, provided that they are different from one another.

For example, Fig. 10 has illustrated the configuration in which the inner coating portion 302a and the outer coating portion 303 have been entirely removed in the circumferential direction of the lead 301a. In a portion of the lead 301a, the inner coating portion 302a and the outer coating portion 303 may be removed only partly in the circumferential direction. The same applies to the leads 301b-301e.

For example, Fig. 10 has illustrated the configuration in which the first conductor 42a has been filled throughout the inner side of the first tube 41a. The first conductor 42a may be positioned only in a portion of the inner side of the first tube 41a. The same applies to the conductors positioned within the second to sixth tubes 41b-41f. For example, in Figs. 11 and 12, the configuration in which the first conductor 43a has been filled throughout the inner side of the first tube 41a and throughout the inner side of the second tube 41b has been illustrated. The first insulator 43a may be partly positioned within the first tube 41a only. The first insulator 43a may be partly positioned within the second tube 41b only. The same applies to the second to fifth insulators 43b-43e.

For example, Fig. 12 has illustrated the configuration in which the second core shaft 12 has passed through the insulators 43a-43e, at the inner side of the connector 40. However, the second core shaft 12 may not pass through the insulators 43a-43e. In this case, the proximal end 122 of the second core shaft 12 is located at the distal end of the connector 40. The third joint part 53 connects the proximal end portion of the second core shaft 12 to the distal end of the connector 40.

### [Modified Example 3]

The configurations of the medical device with a sensor 1, 1A-1E of the first to sixth embodiments, and each configuration of the above modified examples 1, 2 may be combined appropriately. For example, the connector may be configured to incorporate at least a part of features of the connector 40A described in the second embodiment, the connector 40B described in the third embodiment, the connector 40C described in the fourth embodiment, the connector 40D described in the fifth embodiment, and the connector 40E described in the sixth embodiment.

The present aspects have been described above based on the embodiments and modified examples. The embodiments of aspects described above are for ease of understanding of the present aspects, and do not limit the present aspects. The present aspects may be modified or improved without departing from the spirit thereof and claims, and the present aspects include their equivalents. Unless such technical features are explained as essentials in the present specification, they can be appropriately deleted.

## Claims

1. A medical device with a sensor (1, 1A-1E) comprising:
a sensor (20);
wiring (30) electrically connected to the sensor (20);
a conductive first tube (41a) located on the more proximal end side than the sensor (20) and electrically connected to the wiring (30);
a conductive second tube (41b, 41bA-41bC) located on the more proximal end side than the first tube (41a) and electrically connected to the wiring (30); and
an insulator (43a, 43aA, 43aB, 43aD) sandwiched between the first tube (41a) and the second tube (41b, 41bA-41bC), a part of the insulator being externally exposed, wherein a shape of an exposed portion of the insulator when viewed from a first radial direction is different from a shape of the exposed portion of the insulator when viewed from a second radial direction different from the first radial direction.

2. The medical device with a sensor (1, 1A-1E) according to claim 1,
wherein the insulator (43a, 43aA, 43aB, 43aD) is an adhesive.

3. The medical device with a sensor (1, 1A, 1C-1E) according to claim 1 or 2,
wherein a proximal end of the first tube (41a) is located on the more proximal end side than a distal end of the second tube (41b, 41bA, 41bC).

4. The medical device with a sensor (1, 1A-1B, 1D-1E) according to any one of claims 1 to 3,
wherein a virtual cylindrical surface formed by extending an outer peripheral surface of the first tube (41a) to a proximal end side substantially coincides with an outer peripheral surface of the second tube (41b, 41bA-41bB).

5. The medical device with a sensor (1, 1A-1E) according to any one of claims 1 to 4, further comprising:
a core shaft (12) passing through the insulator (43a, 43aA, 43aB, 43aD).

6. The medical device with a sensor (1, 1A-1E) according to any one of claims 1 to 5,
wherein at least any one of a proximal end side end face (412a) of the first tube (41a) and a distal end side end face (411b, 411bA, 411bB) of the second tube (41b, 41bA-41bC) includes a portion inclined with respect to the long axis direction of the medical device with a sensor (1, 1A-1E).

7. The medical device with a sensor (1, 1A, 1C-1E) according to any one of claims 1 to 6,
wherein at least a part of the proximal end side end face (412a) of the first tube (41a) is substantially parallel to at least a part of the distal end side end face (411b, 411bA) of the second tube (41b, 41bA, 41bC).

8. The medical device with a sensor (1, 1A-1C, 1E) according to any one of claims 1 to 7,
wherein at least any one of the first tube (41a) and the second tube (41b, 41bA-41bC) is joined to the insulator (43a, 43aA, 43aB) at an inner peripheral surface.

9. The medical device with a sensor (1, 1A-1D) according to any one of claims 1 to 8,
wherein the insulator sandwiched between the first tube (41a) and the second tube (41b, 41bA-41bC) is a first insulator (43a, 43aA, 43aB,43aD), and
wherein the medical device further comprises:
a conductive third tube (41c) located on the more proximal end side than the second tube (41b, 41bA-41bC) and electrically connected to the wiring (30); and
a second insulator (43b) being sandwiched between the second tube (41b, 41bA-41bC) and the third tube (41c), a part of the second insulator is externally exposed, and a shape of an exposed portion of the second insulator when viewed from the first radial direction is different from a shape of the exposed portion of the second insulator when viewed from the second radial direction.

10. The medical device with a sensor (1, 1A-1D) according to claim 9,
wherein the shape of the exposed portion of the first insulator (43a, 43aA, 43aB, 43aD) is substantially identical with the shape of the exposed portion of the second insulator (43b) when viewed from the first radial direction.
